## Europäisches Patentamt
## European Patent Office
## Office européen des brevets

(19)

(11) Veröffentlichungsnummer: **0 252 258**
**A1**

(12) **EUROPÄISCHE PATENTANMELDUNG**

(21) Anmeldenummer: 87107319.3

(22) Anmeldetag: 20.05.87

(51) Int. Cl.³: **A 61 M 5/00**
**A 61 M 5/31**

(30) Priorität: 31.05.86 DE 3618390

(43) Veröffentlichungstag der Anmeldung:
13.01.88 Patentblatt 88/2

(84) Benannte Vertragsstaaten:
AT CH DE ES FR GB IT LI NL SE

(71) Anmelder: Fresenius AG
Gluckensteinweg 5
D-6380 Bad Homburg(DE)

(72) Erfinder: Walliard, Petra
Rue des Lilas 4
F-57520 Grosbliederstroff(FR)

(74) Vertreter: Görtz, Dr. Fuchs, Dr. Luderschmidt
Patentanwälte
Sonnenberger Strasse 100 Postfach 26 26
D-6200 Wiesbaden(DE)

(54) Injektionsstelle für medizinische Flüssigkeiten.

(57) Eine Injektionsstelle (10) für medizinische Flüssigkeiten weist ein Gehäuse (12) mit einem durchstechbaren Septum oder Membran (18) auf. Die Membran (18) ist als Composite-Membran ausgebildet und besteht aus einer weichen Membranschicht (34) und einer harten Membranschicht (32), die sich in ihrem Shore-Härtegrad unterscheiden.

EP 0 252 258 A1

Croydon Printing Company Ltd

## Beschreibung

Die Erfindung betrifft eine Injektionsstelle für medizinische Flüssigkeiten, die eine Membran mit wenigstens einer durchstechbaren und sich wieder schließenden elastomeren weichen Membranschicht aufweist, die entlang ihres Umfangs in einer Halterung fixiert ist.

Injektionsstellen werden in der Medizin im steigenden Maße sowohl für die extrakorporale als auch die intrakorporale Verabreichung von Medikamenten eingesetzt. Hierbei wird mit Hilfe einer Spritze ein pharmazeutisches Präparat einem steril abgeschlossenen System, beispielsweise einem Katheterschlauch, einer Infusionsflasche oder einem Infusionsbeutel oder auch einer Infusionsleitung zugesetzt. Dabei hat die Membran, die in der Injektionsstelle oder dem Zuspritzteil vorgesehen ist, folgende Anforderungen zu erfüllen:

a) sie muß einerseits so weich in ihrer Shore-Härte sein, daß sie ohne weiteres mehrfach, häufig 1000 mal punktiert werden kann, ohne daß sie dabei irreversibel zerstört wird, und

b) andererseits sich beim Herausziehen der Kanüle unter Beibehaltung der Sterilität, d. h. des Abschlusses der von ihr abgedeckten sterilen Leitung, wieder schließen.

Diese Anforderungen erfüllt im wesentlichen ein relativ weiches Membranmaterial, das sich in Folge seiner hohen elastischen Eigenschaften relativ einfach komprimieren läßt.

Solche Injektionsstellen sind beispielsweise aus dem
DE-GM 77 27 563, dem DE-GM 83 07 992 und dem
DE-GM 84 34 177 bekannt.

Die DE-GM 77 27 563 beschreibt ein Zuspritzgehäuse mit
dichtsitzender Membran für intermittierende Punktionen,
das zur Verbindung mit Infusionsschläuchen oder Kathetern
eingesetzt werden kann. Es besteht im wesentlichen aus
einem rohrförmigen Gehäuse, dessen eine Öffnung mit der
elastischen Membran verschlossen ist, während die andere
Öffnung entweder mit einem Konnektorteil zum Anschluß an
eine Schlauchleitung oder aber direkt mit einer Schlauchleitung verbunden ist.

Die Membran ist in dem rohrförmigen Gehäuse mechanisch
mit Hilfe eines nach innen umgeformten Kragens aus dem
Gehäusematerial unter Andrücken an ein im Gehäuse angebrachtes Membranlager in axialer Richtung entlang ihres
Randes fixiert. Die Membran ist also in axialer Richtung
im Bereich ihres Umfangsrandes lediglich durch den nach
innen geformten Kragen zusammengepreßt und somit lagefixiert.

Die in der DE-GM 77 27 563 beschriebene Zuspritzstelle
weist wenigstens eine relativ große Ausflußöffnung auf,
aus der die eingespritzte Flüssigkeit relativ schnell
abfließen kann, so daß sich innerhalb des rohrförmigen
Gehäuses kein größerer Innendruck aufbauen kann.

Aus der DE-GM 83 07 992 und der DE-GM 84 34 177 sind implantierbare Zuspritzkatheter bekannt, die aus einem Zuspritzgehäuse bestehen, das eine Einlaßöffnung und eine
Auslaßöffnung aufweist. Die Einlaßöffnung ist dabei wiederum mit einer durchstechbaren Membran in der vorstehend
erwähnten Umbördelung des Kragens fixiert, während andererseits die Ausflußöffnung mit einem Katheter verbunden ist, der üblicherweise in ein Blutgefäß implantiert ist.
Derartige Katheterschläuche weisen üblicherweise ein
Lumen mit einem sehr geringen Innenquerschnitt auf, der
den Fluß der Injektionslösung limitiert. Infolge der Inkompressibilität der zugeführten Injektionslösung kann
beim Injizieren einer solchen Flüssigkeit mit einer Spritze folgender Zustand auftreten:
Das Innenvolumen des Zuspritzgehäuses ist wegen der Implantierbarkeit der gesamten Anordnung relativ klein gehalten. Wird nunmehr aus einer größer kalibrierten Spritze eine Injektionslösung relativ schnell zugeführt, so
kann sich innerhalb des Gehäuses infolge der Inkompressibilität der Lösung und der relativ geringen Ausströmrate durch den Katheterschlauch ein relativ großer Innendruck innerhalb des Gehäuses aufbauen, der 3 und mehr bar
betragen kann. Da die Membran entlang ihres Umfangrandes
nur mechanisch zusammengepreßt ist, kann es infolge des
hohen Innendrucks zu einem Herausziehen der Membran aus
der Fixierungsstelle kommen, so daß die injizierte Flüssigkeit unmittelbar in das umgebende Gewebe abfließen
kann, was höchst unerwünscht ist. So konnte festgestellt
werden, daß die üblicherweise für derartige Injektionsstellen eingesetzten Membranen bereits bei Drücken von
3 bar aus ihrer Halterung gerissen worden sind, was zu
den vorstehend erwähnten unerwünschten Folgeerscheinungen
führte.

Der Erfindung liegt daher die Aufgabe zugrunde, die Injektionsstelle der eingangs erwähnten Art so fortzubilden, daß sie auch bei Drücken von 3 bar und mehr ohne
Gefahr für den Patienten gehandhabt werden kann.

Die Lösung der Aufgabe erfolgt dadurch, daß die weiche
Membranschicht mit wenigstens einer eine größere Shore-
Härte aufweisenden harten Membranschicht verbunden ist.

Die erfindungsgemäße Vorrichtung weist zunächst den Vorteil auf, daß sie wesentlich höhere Drücke aushält als die bekannte Ausführungsform, die lediglich eine relativ weiche und elastische Membran aufweist. So konnten der erfindungsgemäßen Injektionsstelle Injektionslösungen mit einem Druck von mehr als 6 bar zugeführt werden, ohne daß sich die Membran aus ihrer Halterung löste.

Hierdurch wird insbesondere die Handhabbarkeit von implantierbaren Katheterinfusionssystemen verbessert, bei denen die Injektionsstelle mit einem Katheter verbunden ist, der einen relativ geringen Lumenquerschnitt aufweist.

Die erfindungsgemäß eingesetzte Membran weist somit folgende Vorteile auf:
Der weiche Membranteil ist für relativ viele Punktionen geeignet und verschließt sich nach jeder Punktion selbsttätig unter Beibehaltung der Sterilität des Innenraums der Injektionsstelle.

Die harte Membran stabilisiert den Sitz der weichen Membran, d.h. verhindert ein Aufblähen der gesamten Membrananordnung infolge zu starken Innendrucks. Andererseits kommen jedoch die Nachteile dieser Membran (nur geringe Punktionsfähigkeit und unter Umständen mangelhafte Sterilität nach der Punktion) nicht zum Tragen, da die weiche Membran diese Nachteile auffängt.

Erfindungsgemäß liegt das Verhältnis der Shore-Härte zwischen der harten und der weichen Membranschicht zwischen etwa 3 : 1 und etwa 1,2 : 1, vorzugsweise zwischen etwa 2 : 1 und 1,5 : 1.

Die Shore-Härte selbst bestimmt sich nach der DIN 53 501. Dabei wird die Härte der gummielastischen Membranschicht mit einem Kegelstumpf oder einem abgerundeten Kegel als Eindringkörper ermittelt. Der Eindringkörper wird bis zum Aufliegen der Auflagefläche des Gerätes in die Probenfläche gedrückt und dabei eine Druckfeder gespannt. Erfindungsgemäß wird die Shore-Härte in den Werten A gemäß DIN 53 505, Ausgabe 8.73, angegeben.

Die Shore-Härte der weichen Membranschicht nimmt üblicherweise den Wert A 30 - A 50, vorzugsweise den Wert von etwa A 40 ein.

Dagegen beträgt die Shore-Härte der harten Membranschicht etwa A 60 - 90, vorzugsweise etwa A 70.

In welcher Reihenfolge erfindungsgemäß die Anordnung der harten und der weichen Membranschicht gewählt wird, ist dem Fachmann überlassen. Vorteilhafterweise wird man eine zweischichtige Anordnung, die aus der weichen und der harten Membranschicht besteht, so anordnen, daß die weiche Membranschicht dem Innenraum des Gehäuses der Injektionsstelle zugewandt ist, während die harte Membranschicht der Umgebung zugewandt ist. Andererseits kann jedoch auch, sofern der Einsatzzweck dies notwendig macht, die harte Membranschicht innen liegen, während die weiche Membranschicht außen angeordnet ist.

Die Anwendung einer derartigen Composite-Membran ist jedoch nicht auf eine zweischichtige Anordnung beschränkt, vielmehr können jedoch auch drei- und mehrschichtige Anordnungen eingesetzt werden, sofern eine höhere Stabilität erwünscht ist.

Gemäß einer weiteren bevorzugten Ausführungsform kann eine dreischichtige Anordnung gewählt werden, bei der die weiche Membranschicht sandwichartig zwischen zwei harten Membranschichten angeordnet ist.

In einer anderen Ausführungsform kann jedoch aber auch eine harte Membranschicht sandwichartig zwischen zwei weichen Membranschichten angeordnet sein.

Üblicherweise werden dabei die vorstehend erwähnten Shore-Härtegrade für die Membranschichten eingesetzt, wobei vorzugsweise die außenliegenden Schichten etwa die gleiche Shore-Härte aufweisen.

Als Material für derartige Membranschichten werden die üblicherweise eingesetzten, für den medizinischen Gebrauch zugelassenen, Membranwerkstoffe eingesetzt, beispielsweise Gummi, Silikonkautschuk, Chlorbutylkautschuk und dergl. Vorzugsweise wird erfindungsgemäß Silikonkautschuk wegen seiner inerten Eigenschaften eingesetzt. So ist insbesondere ein Silikonkautschuk bevorzugt, der nicht mit Füllstoffen gefüllt ist. Ein derartiges Silikonmaterial kann in den oben angegebenen Shore-Härten ohne Schwierigkeiten hergestellt und in Form von medizinisch unbedenklichem Material zur Verfügung gestellt werden.

Die harte und die weiche Membranschicht werden vor der Montage im Zuspritzteil vorteilhafterweise miteinander verbunden, wobei gemäß einer ersten Ausführungsform die einzelnen Membranschichten mittels einer Klebeschicht miteinander verklebt werden. Sofern ein Silikonkautschuk für die Membranschichten gewählt worden ist, wird vorteilhafterweise ein mit dem Silikonkautschuk verträglicher Silikonkleber eingesetzt. Für andere Materialien, beispielsweise Gummi, wird ein üblicher Vulkanisierkleber verwendet.

Andererseits kann jedoch aber auch die erfindungsgemäß einzusetzende Membrananordnung bereits werkseitig als integrale Einheit hergestellt werden. Hierbei werden die Schichten unterschiedlichen Shore-Härtegrades während der Polymerisationsphase in Berührung gebracht, so daß ein einheitliches Polymerisationsgebilde entsteht.

Solchermaßen hergestellte Membranschichten können nach dem üblichen Stanzverfahren zu den zu montierenden Membranstücken verarbeitet werden, die in das Zuspritzgehäuse eingesetzt und dort entlang ihres Randes mit dem Gehäuse, vorzugsweise durch Umformung des Kragens, verspannt wird. Hinsichtlich dieses Befestigungsverfahrens wird auf das DE-GM 77 27 563 verwiesen.

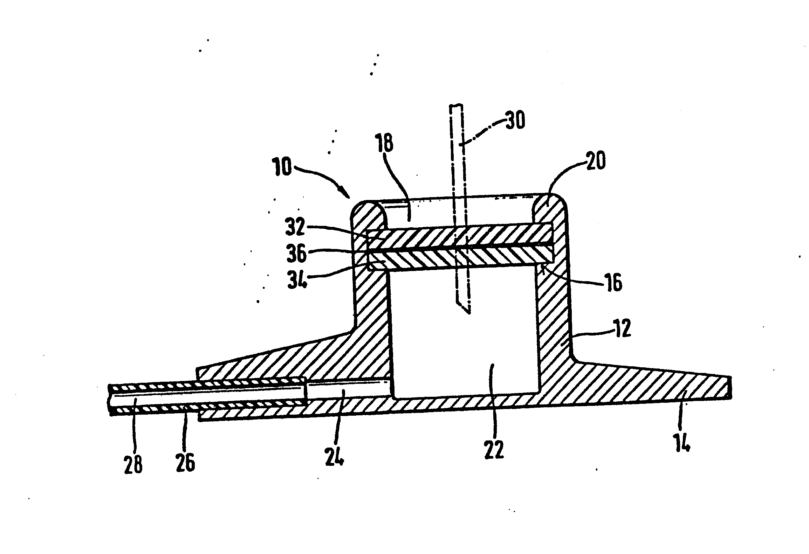

Weitere Merkmale, Einzelheiten und Vorteile der Erfindung werden nachfolgend anhand eines Ausführungsbeispiels unter Bezugnahme auf die Zeichnung erläutert.

Es zeigt:
die Figur einen senkrechten Mittelschnitt durch eine erste Ausführungsform einer implantierbaren Injektionsstelle im vergrößerten Maßstab.

Die in der Figur gezeigte Injektionsstelle (10), die in den Körper eines Patienten implantiert werden kann, besteht aus einem rohrförmigen Gehäuse (12), das an seiner Unterseite zu einem Flansch (14) verbreitert ist.

Auf der Innenseite weist das Gehäuse (12) eine ringförmige Ausnehmung (16) auf, die als Lagerfläche für eine durchstechbare Membran (18) dient. Diese Membran (18) ist entlang ihres Umfangsrandes durch einen nach innen gebogenen Kragen (20) des Gehäuses (12) gegen die Lagerfläche der Ausnehmung (16) gedrückt und wird hierdurch lagefixiert.

Das Gehäuse (12) weist weiterhin einen Innenraum (22) auf, der vollständig durch die Membran (18) abgeschlossen wird und der mit einem Ausgangsstutzen (24) in Strömungsverbindung steht, der in Form einer Bohrung den Flansch (14) radial durchsetzt.

In diesen Ausgangsstutzen (24) ist das Ende eines Katheterschlauchs (26) eingelassen. Infolgedessen steht der Innenraum (22) mit dem Lumen (28) des Katheterschlauchs (26) über den Ausgangsstutzen (24) in Strömungsverbindung.

Wie in der Figur strichliert dargestellt ist, kann die Membran (18) mit einer Injektionskanüle (30) durchstochen werden, so daß der Inhalt einer Spritze in den Innenraum (22) und von dort durch den Anschlußstutzen (24) in den Katheterschlauch befördert werden kann.

Die Membran (18) der Injektionsstelle (10) besteht aus einer relativ harten Membranschicht (32) und einer im Vergleich hierzu relativ weichen Membranschicht (34), die gemäß der in der Figur gezeigten Ausführungsform mittels einer Klebeschicht (36) miteinander verbunden sind.

Die weiche Membranschicht (34) weist eine Shore-Härte von A 35 - A 50 auf, während die harte Membranschicht (32) eine Shore-Härte von etwa A 60 - A 80 besitzt.

Das Membranmaterial besteht jeweils aus einem Silikonkautschuk, während für die Klebeschicht (36) ein Silikonkleber eingesetzt worden ist.

Die Dicke der weichen bzw. der harten Membranschicht liegt in einem Bereich von etwa 1,8 - 2 mm, während die Klebeschicht möglichst gering gewählt ist und nur zu Verbindungszwecken eingesetzt wird.

Die Membran (18) läßt sich in einer Vielzahl von Punktierungen durchstechen, ohne daß hierunter der Punktierungsgrad und die Sterilität der Injektionsstelle (10) leidet. Diese Eigenschaft ist auf die weiche Membranschicht (34) der Membran (18) zurückzuführen.

Andererseits ist die Druckbeständigkeit der Membrananordnung auf die harte Membranschicht (32) zurückzuführen, die auch bei Drücken oberhalb 3 bar die Membran (18) stabil in ihrem Sitz hält. Dabei ist es nicht nachteilig, wenn die Membran einer Vielzahl von Funktionen unterzogen wird, d. h. auch nach einer derartigen Behandlung bleibt die Membran fest mit dem Gehäuse verhaftet, selbst wenn im Zentralbereich, in dem üblicherweise die Punktion stattfindet, die harte Membranschicht (32) durch die Punktionen weitgehend ihre Festigkeit verloren hat.

-.-

Patentansprüche

1. Injektionsstelle für medizinische Flüssigkeiten, die
   eine Membran mit wenigstens einer durchstechbaren und
   sich wieder schließenden elastomeren weichen Membranschicht aufweist, die entlang ihres Umfangs in einer
   Halterung fixiert ist, d a d u r c h  g e k e n n -
   z e i c h n e t, daß die weiche Membranschicht (34)
   mit wenigstens einer eine größere Shore-Härte aufweisenden harten Membranschicht (32) verbunden ist.

2. Injektionsstelle nach Anspruch 1, d a d u r c h  g e -
   k e n n z e i c h n e t, daß das Verhältnis der Shore-
   Härte zwischen der harten Membranschicht (32) und der
   weichen Membranschicht (34) zwischen 3 : 1 und
   1,2 : 1, vorzugsweise 2 : 1 und 1,5 : 1 liegt.

3. Injektionsstelle nach Anspruch 1 oder 2, d a d u r c h
   g e k e n n z e i c h n e t, daß die Shore-Härte der
   weichen Membranschicht (34) den Wert A 30 - A 50, vorzugsweise etwa A 40 aufweist.

4. Injektionsstelle nach Anspruch 1 oder 2,
   d a d u r c h  g e k e n n z e i c h n e t, daß
   die Shore-Härte der harten Membranschicht (32) den
   Wert A 60 - A 90, vorzugsweise etwa A 70 aufweist.

5. Injektionsstelle nach einem der Ansprüche 1 bis 4,
   d a d u r c h  g e k e n n z e i c h n e t, daß die
   harte Membranschicht (32) der Umgebung und die weiche
   Membranschicht (34) dem Innenraum (22) eines Gehäuses
   (12) zugewandt ist.

6. Injektionsstelle nach einem der Ansprüche 1 bis 4, d a d u r c h   g e k e n n z e i c h n e t, daß die Membran außer der weichen und der harten Membranschicht eine weitere weiche dritte Membranschicht aufweist, und die harte Membranschicht zwischen den beiden weichen Membranschichten angeordnet ist.

7. Injektionsstelle nach einem der Ansprüche 1 bis 4, d a d u r c h   g e k e n n z e i c h n e t, daß die Membran außer der weichen und der harten Membranschicht eine weitere harte dritte Membranschicht aufweist und die weiche Membranschicht zwischen den harten Membranschichten angeordnet ist.

8. Injektionsstelle nach einem der Ansprüche 1 bis 7, d a d u r c h   g e k e n n z e i c h n e t, daß das Material der Membran (18) Silikonkautschuk, Gummi oder Latex ist.

9. Injektionsstelle nach einem der Ansprüche 1 bis 8, d a d u r c h   g e k e n n z e i c h n e t, daß die benachbarten Membranschichten jeweils durch eine Klebeschicht miteinander verbunden sind.

10. Injektionsstelle nach einem der Ansprüche 1 bis 8, d a d u r c h   g e k e n n z e i c h n e t, daß die benachbarten Membranschichten während der Polymerisationsphase aneinander vulkanisiert worden sind und eine integrale Membran bilden.

-.-

0252258

### Europäisches Patentamt

## EUROPÄISCHER RECHERCHENBERICHT

Nummer der Anmeldung

| | EINSCHLÄGIGE DOKUMENTE | | EP 87107319.3 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | US - A - 4 190 040 (R.R. SCHULTE)<br>* Gesamt *<br>-- | 1 | A 61 M 5/00<br>A 61 M 5/31 |
| A | CH - A - 485 463 (SCHERICO LTD)<br>* Gesamt; insbesondere Spalte 3, Zeile 65 - Spalte 4, Zeile 30 *<br>-- | 1 | |
| A | EP - A2 - 0 142 866 (CONSOLIDATED CONTR.)<br>* Fig. 5; Seite 10, Zeilen 16-28 *<br>-- | 1 | |
| A | DE - A1 - 2 716 447 (M. FAENSEN)<br>* Fig. 1,2,4,5; Patentansprüche 1-4 *<br>-- | 1 | RECHERCHIERTE SACHGEBIETE (Int. Cl 4) |
| D,A | DE - U1 - 8 307 992 (K. AIGNER)<br>* Gesamt *<br>-- | 1 | A 61 M 5/00<br>A 61 M 25/00 |
| D,A | DE - U1 - 8 434 177 (B. BRAUN MELS. AG)<br>* Gesamt *<br>-- | 1 | |
| D,A | DE - U1 - 7 727 563 (B. BRAUN MELS. AG)<br>* Gesamt *<br>-- | 1 | |

Der vorliegende Recherchenbericht wurde für alle Patentansprüche erstellt.

| Recherchenort<br>WIEN | Abschlußdatum der Recherche<br>16-10-1987 | Prüfer<br>LUDWIG |
|---|---|---|

EPA Form 1503. 03 82

**KATEGORIE DER GENANNTEN DOKUMENTEN**
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches
Patentamt

| | EINSCHLÄGIGE DOKUMENTE | | EP 87107319.3 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| P,A | DE - A1 - 3 518 841 (SIEMENS AG)<br><br>  \* Gesamt; insbesondere Fig.;<br>    Zusammenfassung \*<br><br>       ----  | 1 | |
| | | | **RECHERCHIERTE SACHGEBIETE (Int. Cl.4)** |

Der vorliegende Recherchenbericht wurde für alle Patentansprüche erstellt.

| Recherchenort | Abschlußdatum der Recherche | Prüfer |
|---|---|---|
| WIEN | 16-10-1987 | LUDWIG |

KATEGORIE DER GENANNTEN DOKUMENTEN

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
    nach dem Anmeldedatum veröffentlicht worden ist
D : in der Anmeldung angeführtes Dokument
L : aus andern Gründen angeführtes Dokument

& : Mitglied der gleichen Patentfamilie, überein-
    stimmendes Dokument

EPA Form 1503 03 82